# EUROPÄISCHE PATENTSCHRIFT

(11) **EP 0 782 869 B1**
(45) Veröffentlichungstag und Bekanntmachung des Hinweises auf die Patenterteilung: **24.04.2002**
(21) Anmeldenummer: 96118660.8
(22) Anmeldetag: 21.11.1996
(51) Int. Cl.: A61M 35/00, D06M 23/12

(54) **Mikrokapselbeschichtetes Material**
Material coated with micro-capsules
Materiel revêtu avec des micro-capsules

(30) Priorität: 03.01.1996 DE 19600076
(43) Veröffentlichungstag der Anmeldung: 09.07.1997
(73) Patentinhaber: Deotexis Inc., New York, N.Y. 10022-4838 (US)
(72) Erfinder: Tebbe, Gerold, 9800 Monaco (MC)
(74) Vertreter: Ostertag, Reinhard

(56) Entgegenhaltungen:
- EP-A- 0 328 937
- DE-A- 3 545 926
- US-A- 3 896 807
- US-A- 4 567 065

## Beschreibung

Die Erfindung betrifft ein mikrokapselbeschichtetes, flexibles Trägermaterial gemäß dem Oberbegriff des Anspruchs 1.

Ein solches Trägermaterial ist in der DE 34 47 833 A beschrieben.

In der US-A-4 567 065 ist ein Handschuh und eine Manschette beschrieben, welche auf ihrer Innenseite mit einem Pflegemittel oder einem Heilmittel beschichtet sind. Auf diese Weise kann eine Behandlung von Hautstellen erfolgen, ohne daß das aufgetragene Mittel versehentlich andere Gegenstände verunreinigt oder unabsichtlich abgewischt wird.

In der DE 35 45 926 A ist ein Tuch beschrieben, welches einen Wirkstoff enthaltende Kapseln enthält. Diese Kapseln haben makroskopische Abmessungen und sind in die Verbundstruktur des Tuchs integriert, welches neben einem Vlies oder dergleichen eine für das verkapselte Mittel undurchlässige Trennschicht aufweist.

Wenn die mikrokapselbeschichtete Seite eines Trägermateriales der eingangs angesprochenen Art, insbesondere in Form eines Tuches, mit Druck auf einer Oberfläche gerieben wird, brechen die Mikrokapseln auf, und das darin enthaltene Material wird freigesetzt und auf die Oberfläche aufgebracht.

Bei Tüchern mit einer Mikrokapselbeschichtung bleiben erfahrungsgemäß große Teile des Tuchs unbenutzt, da Aufreiben unter Druck nur an bestimmten Stellen des Tuchs vorgenommen wird. Damit bleibt auch ein großer Teil des teuren mikroverkapselten Materials ungenutzt. Weiter ist, insbesondere wenn die Oberfläche, auf die das mikroverkapselte Material aufzubringen ist, eine größere Neigung zur Horizontalen aufweist, eine besondere Aufmerksamkeit erforderlich, um den Kontakt zwischen Hand und Tuch aufrechtzuerhalten. Außerdem kann die Verwendung eines mikrokapselbeschichteten Tuchs dazu führen, daß verkapseltes Material versehentlich an einer Stelle aufgebracht wird, wo dies nicht erwünscht ist, nämlich dann, wenn auf Teile des Tuchs, die eine solche Stelle bedecken, versehentlich Druck ausgeübt wird, z.B. weil versucht wird, das Tuch am Herunterfallen zu hindern.

Aufgabe der Erfindung ist es demnach, ein flexibles Trägermaterial mit einer Mikroverkapselbeschichtung in einer Form zur Verfügung zu stellen, die einfacher handhabbar ist und eine bessere Ausnutzung des mikroverkapselten Material erlaubt.

Diese Aufgabe wird erfindungsgemäß durch ein mikrokapselbeschichtetes, flexibles Trägermaterial gemäß Anspruch 1 gelöst.

Für viele Anwendungen ist es vorteilhaft, wenn das flexible Trägermaterial mit der Mikrokapselbeschichtung in der Art eines Handschuhs festsitzend über die Hand oder einen Teil der Hand gezogen werden kann. Damit wird eine optimale Ausnutzung des Mikrokapselbeschichtung sowie einfachste Handhabbarkeit ermöglicht, da mit der Hand auf alle Stellen des Handschuhs, auf denen die Mikrokapselbeschichtung angebracht ist, ohne weiteres Druck ausgeübt werden kann und der feste Kontakt zwischen Trägermaterial und Hand automatisch gewährleistet ist. Weiter ist es mit einem solchen Trägermaterial in Form eines Handschuhs möglich, versehentliches Aufbringen des mikroverkapselten Materials an Stellen, wo dies unerwünscht ist, zu vermeiden, da durch den festen Sitz des Handschuhs die Position der Stelle, auf die Druck ausgeübt wird, genau gesteuert werden kann und versehentliches Druckausüben, z.B. infolge eines Auffangversuches des herunterfallenden Trägermaterials, vermieden wird.

Vorteilhafte Weiterbildungen der Erfindung sind in den Unteransprüchen angegeben.

Die Weiterbildung der Erfindung gemäß Anspruch 2 ist im Hinblick auf eine vereinfachte Herstellung in wenigen Größen von Vorteil.

Die Weiterbildung der Erfindung gemäß Anspruch 3 erlaubt eine abfallose Herstellung des Handschuhs.

Die Weiterbildung gemäß Anspruch 4 gestattet eine zusätzliche Trägermaterialeinsparung.

Die Weiterbildung der Erfindung gemäß Anspruch 5 erlaubt die Herstellung des Handschuhs in einer einzigen Größe, der im Bedarfsfalle entsprechend der Größe der Hand des Benutzers durch ein Mittel zum Zusammenziehen in einem Abschnitt zusammengezogen werden kann und so einen festen Sitz auf der Hand gewährleistet.

Die Weiterbildung gemäß Anspruch 6 stellt durch ein Mittel zum Ankleben an der Haut einen besonders festen Sitz sicher.

Die Weiterbildug gemäß Anspruch 7 vereinfacht das Anbringen der Mittel zum Zusammenziehen und zum Ankleben an der Haut am Trägermaterial.

Die Weiterbildung gemäß Anspruch 8 sorgt für eine optimale Ausnutzung der Mikrokapselbeschichtung.

Auch die Weiterbildung gemäß Anspruch 9 ist bezüglich einer sparsamen Verwendung des mikroverkapselten Materials, insbesondere beim Aufbringen auf einer konvexen Oberfläche, von Vorteil.

Die Weiterbildung gemäß Anspruch 10 erweist sich insbesondere beim Aufbringen auf einer im wesentlichen planen Oberfläche als vorteilhaft.

Die Weiterbildung gemäß Anspruch 11 ist vor allem dann von Vorteil, wenn die Fingerspitzen während des Aufbringens des mikroverkapselten Materials eine zusätzliche Aufgabe erfüllen sollen, z.B. das Mitführen eines Poliertuches beim Aufbringen eines Politurmittels. Auch kann man einen solchen Handschuh von beiden Enden her anziehen, wenn er rechteckige Form hat.

Mit der Weiterbildung der Erfindung gemäß Anspruch 12 wird erreicht, daß die Mikrokapseln tragende Oberfläche des Handschuhs besonders groß ist. Der aufgerauhte oder flauschige Charakter der Handschuhaußenseite ist auch im Hinblick auf ein gutes flächendeckendes Verwischen des nach Aufbrechen der Mikrokapseln freigegebenen flüssigen Kapselinhaltes auf der Hautoberfläche von Vorteil.

Die Weiterbildung der Erfindung gemäß Anspruch 13 gestattet es, eine sehr stark aufgerauhte oder flauschige Außenschicht zu verwenden, die keine sichere Sperrfunktion übernehmen kann.

Die Ansprüche 14 und 15 geben bevorzugte Anwendungen der Erfindung auf dem Gebiet der Heilmittel an.

Mit der Weiterbildung der Erfindung gemäß Anspruch 16 wird erreicht, daß die in den Mikrokapseln eingeschlossenen Wirkstoffe nacheinander freigegeben werden. Dies ermöglicht es zum einen, unterschiedliche Wirkstoffe in dem Mikrokapselgemisch einzukapseln, die dann in vorgegebener Reihenfolge (z.B. bei Anwendung nur leichten Druckes und anschließend bei Anwendung höheren Druckes) freigegeben werden. Man kann so z.B. die Hautoberfläche zunächst einölen und anschließend dann mit einem Wirkstoff einreiben, der an der Hautoberfläche wirken soll, jedoch nicht ins Innere der Haut einzudringen braucht (bzw. dort nicht eindringen soll).

Nachstehend wird die Erfindung anhand von Ausführungsbeispielen unter Bezugnahme auf die Zeichnung näher erläutert. In dieser zeigen:
- Figur 1:: die Handinnenseite eines mikrokapselbeschichteten, flexiblen Handschuhs;
- Figur 2:: eine Seite eines doppellagigen Endlosmaterialbands, aus dem einzelne Handschuhe abgetrennt werden können, wobei Nähte und Mikrokapselbeschichtung bereits angebracht sind;
- Figur 3:: die Handinnenseite eines Fausthandschuhs, dessen oberes Drittel mit Mikrokapseln beschichtet ist und der ein Mittel zum Zuziehen am Handgelenk aufweist;
- Figur 4:: die Handinnenseite eines Handschuhs aus flexiblem Trägermaterial, der die Form einer rechteckigen Tasche hat;
- Figur 5:: die Handinnenseite eines Handschuhs in Form einer rechteckigen Tasche, der lediglich über Zeige-, Mittel-, Ringfinger und kleinen Finger gezogen wird;
- Figur 6:: die Oberseite eines doppellagigen Endlosmaterialbands, aus dem die rechteckigen Handschuhe nach Figur 5 abtrennbar sind;
- Figur 7:: einen vergrößerten Schnitt durch einen Bereich eines ersten Trägermateriales für die Verwendung in einem mikrokapselbeschichteten Handschuh;
- Figuren 8 und 9:: ähnliche Schnitte wie Figur 7, in welchen abgewandelte Trägermaterialien gezeigt sind; und
- Figur 10:: einen nochmals vergrößerten Schnitt durch einen Teilbereich einer Beschichtungsschicht der Trägermaterialien von Fig. 7 bis 9.

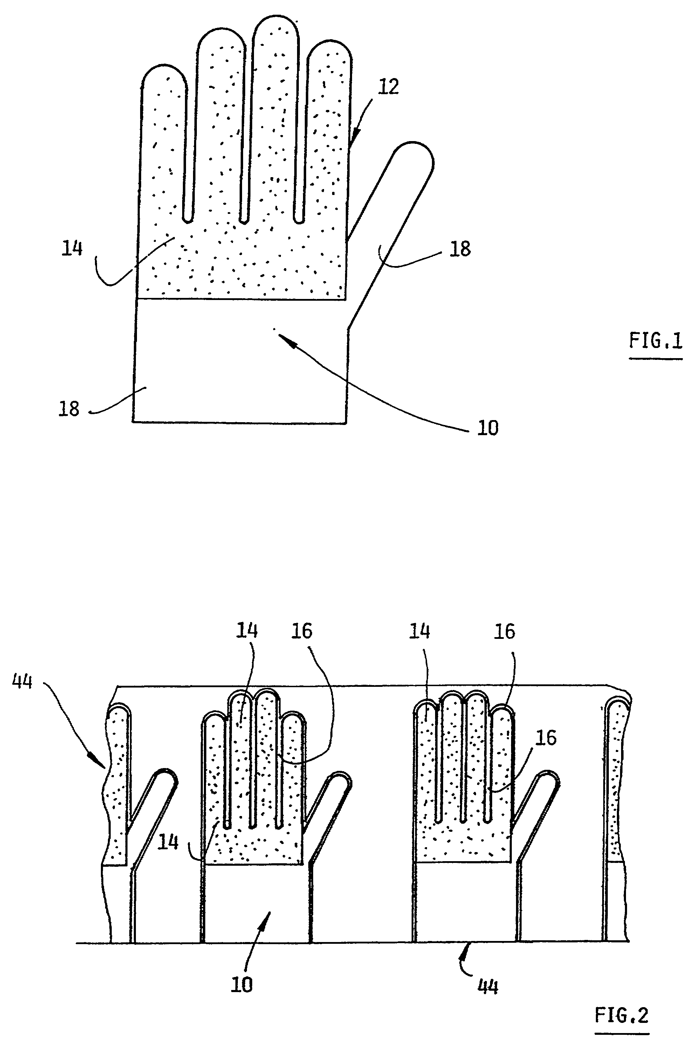

Fig. 1 zeigt die Handinnenseite 10 eines Handschuhs 12, der teilweise eine Beschichtung 14 aus Mikrokapseln trägt. Zugleich Nähte 16 bildende Trennschweißungen verbinden zwei übereinanderliegenden Lagen eines flexiblen Trägermaterials 18.

Als flexibles Trägermaterial 18 kommen alle Materialien in Betracht, die sich mit Mikrokapseln 14 beschichten lassen, die unter Druck aufbrechen. Beispiele dafür sind schweißbare, Bindemittel annehmende Folien, z.B. durch Coronaentladung mit aufgerauhter Oberfläche versehene Polyethylenfolien. Besonders bevorzugt sind Materialien, die Vlies, z.B. Cellulosevlies, mit thermoplastischen Fasern enthalten, da sich Doppelschichten solchen Vlieses einerseits leicht zu Handschuhen 12 verschweißen lassen, Vliese andererseits eine offene Struktur haben und so Mikrokapseln leicht und in größerer Menge annehmen. Außer durch Verschweißen können die Nähte 16 des Handschuhs 12 jedoch auch auf andere Art, z.B. durch Nähen oder Kleben, hergestellt werden.

Fig. 2 zeigt ein doppellagiges Endlosmaterialband 44 für Handschuhe 12 nach Fig. 1.

Das Vereinzeln der zunächst als Endlosband 44 vorliegenden Handschuhe 12 kann bei Trennschweißungen durch Auseinanderziehen, ansonsten z.B. mittels einer Schere, einer Stanzform, eines heißen Messers oder eines Laserstrahls erfolgen.

Die Mikrokapsel-Beschichtung 14 wird vor oder nach der Herstellung der Nähte 16, zweckmäßigerweise vor dem Vereinzeln der Handschuhe 12, aufgebracht. Sie kann auf dem ganzen Handschuh oder nur auf Teilen davon aufgebracht sein. Besonders bevorzugt ist ein Aufbringen auf den oberen zwei Dritteln des Handschuhs oder Teilen davon, z.B. auf dem obersten oder dem mittleren Drittel des Handschuhs oder dessen oberer Hälfte.

Mikrokapseln können ganz allgemein durch Aufkleben oder durch Aufdrücken auf einem Trägermaterial befestigt werden. Bei dem letztgenannten Verfahren muß der Druck jedoch begrenzt werden, um ein Aufbrechen der Mikrokapseln zu vermeiden. So können Gelatinekapseln beispielsweise mittels in einem leicht flüchtigen Lösungsmittel gelöster Gelatine aufgeklebt werden, oder sie können mit noch klebriger oder wieder klebrig gemachter Oberfläche aufgewalzt werden.

Beispiele für mikroverkapselte Materialien, bei denen ein Trägermaterial in Handschuhform besonders nützlich ist, sind Mittel für Schutz- und Imprägnierschichten (zur Vermeidung von Überdosierung), z.B. Schuhputzcreme (genaues und dosiertes Auftragen wird ermöglicht) oder Einfettmittel für technische Bauteile (um teuren Überschuß zu vermeiden), Mittel zur Körperpflege, z.B. Deodorant oder Parfum (zum genau dosierten Auftragen) und perkutane Arzneimittel (ebenfalls zum genau dosierten Auftragen).

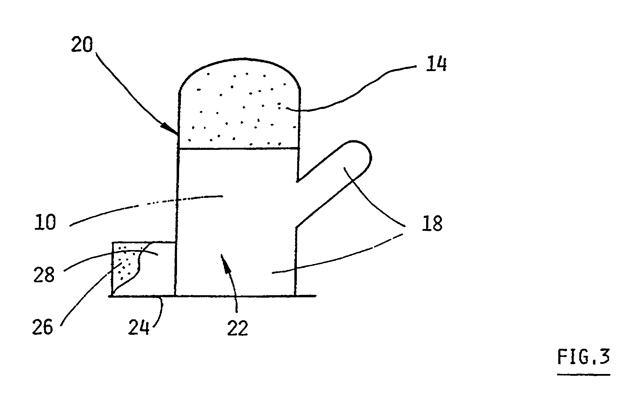

Fig. 3 zeigt die Handinnenseite 10 eines als Fausthandschuh 20 ausgebildeten flexiblen Trägermaterials 18, dessen oberes Drittel eine Mikrokapsel-Beschichtung 14 trägt und der eine Zuziehlasche 22 beim Handgelenk aufweist. Die Zuziehlasche 22 ist in der Regel so am unteren Rand des Handschuhs 20 angebracht, daß sie mit diesem abschließt, muß dies aber nicht. Auf jeden Fall weist sie einen seitlich über den rechten oder linken Rand des Handschuhs 20 hinausragenden Teil 24 auf.

Die Zuziehlasche 22 kann vor oder nach dem Vereinzeln der Handschuhe 20 angebracht werden. Alle Arten der Anbringung sind möglich, z.B. Annähen, Anschweißen, Ankleben; besonders bevorzugt ist Ankleben. Der überstehende Teil 24 der Zuziehlasche trägt zum Festlegen am Handschuh 20 nach dem Zuziehen eine Kleberschicht 26 , deren Klebstoff so beschaffen ist, daß sich der Klebeverschluß nach Gebrauch wieder öffnen läßt. Die Kleberschicht 26 ist vor Gebrauch durch eine abziehbare Abdeckung 28 geschützt.

Andere Arten der Festlegung der Zuziehlasche am Handschuh sind je nach Art des Trägermateriales möglich, z.B. ein Klettverschluß.

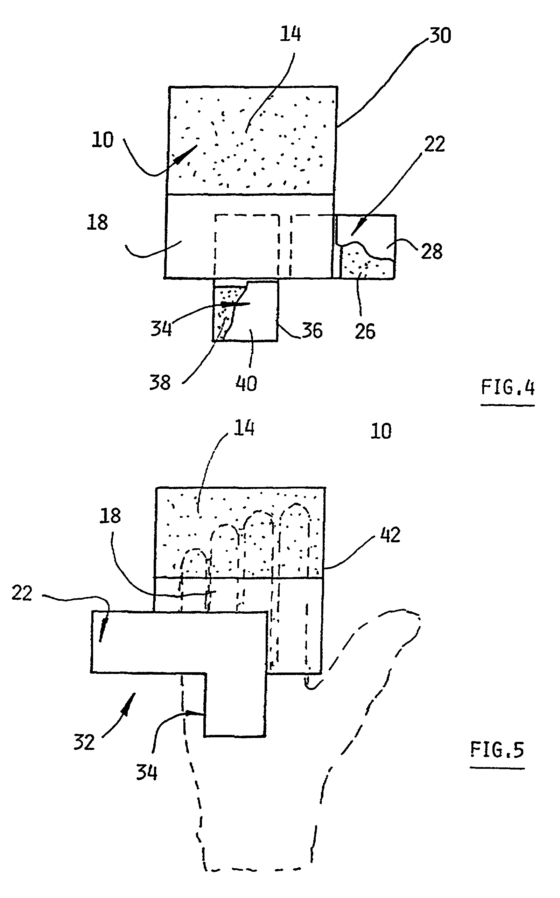

Fig. 4 zeigt einen rechteckigen Handschuh 30, dessen obere Hälfte der Handinnenseite 10 eine Beschichtung 14 mit Mikrokapseln aufweist. Auf der Außenseite des rechteckigen Handschuhs 30 ist eine Zuziehlasche 22 und eine Festkleblasche 34 zum Ankleben an der Haut angeordnet. Die Festkleblasche 34 weist auf einem über die Randkontur des Handschuhs überstehenden Teil 36 eine hautfreundliche Kleberschicht 38 auf, wie dies ähnlich von Heftpflastern bekannt ist. Diese ist vor Gebrauch durch eine abziehbare Abdeckung 40 geschützt. Die Zuziehlasche 22 ist wie bei Fig. 3 beschrieben ausgebildet.

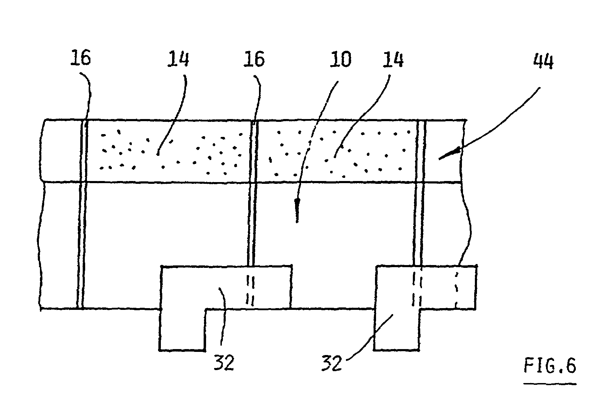

Fig. 5 zeigt einen rechteckigen Handschuh 42, dessen Länge so bemessen ist, daß er über Zeige-, Mittel-, Ringfinger und kleinen Finger ziehbar ist und oberhalb des Daumenansatzes endet. Die obere Hälfte der Handinnenseite 10 des rechteckigen Handschuhs 42 trägt eine Mikrokapsel-Beschichtung 14. Außerdem ist auf der Handinnenseite 10 des Handschuhs 42 eine abgewinkelte Kombilasche 32 vorgesehen, deren über den Handschuhrand überstehende Teile zum Zuziehen des Handschuhs bzw. zu seinem Ankleben an der Haut dienen.

Fig. 6 zeigt die eine Seite eines Endlosmaterialbands 44, die nach dem Vereinzeln der Handfschuhe die Handinnenseiten 10 von rechteckigen Handschuhen 30 oder 42 bildet.

Die Nähte 16 und die Mikrokapsel-Beschichtung 14 sind bereits angebracht. Die Kombilaschen 32 sind vor dem Vereinzeln der Handschuhe auf dem Endlosmaterialband 44 angebracht. Sie können jedoch auch erst nach dem Vereinzeln der Handschuhe 30 bzw. 42 angebracht werden. Die rechteckige Handschuhform ermöglicht eine abfallose Verwendung des Endlosmaterialbands 44 und gestattet einfachste Aufbringung der Mikrokapsel-Beschichtung 14, da keine Leerräume zu beachten sind.

In Abwandlung der Ausführungsbeispiele nach den Fig. 1 bis 5 kann man die Handschuhe auch so ausbilden, daß sie am fingerseitigen Ende offen sind. Bei den Ausführungsbeispielen nach Fig. 4 und 5 nehmen sie demgemäß Hülsenform an.

Bei den Ausführungsbeispielen nach Fig. 1 bis 5 können entweder die Handinnenseite 10 allein oder sowohl die Handinnenseite 10 als auch die Handaußenseite (nicht gezeigt) der Handschuhe mit Mikrokapseln beschichtet sein. Im letzteren Fall kann sich die Mikrokapsel-Beschichtung 14 ebenfalls nur auf einen Teil der jeweiligen Seite erstrecken, wobei die beschichteten Teile auf den beiden Seiten verschieden groß sein können. So können beispielsweise die oberen zwei Dritteln oder die obere Hälfte der Handinnenseite 10 beschichtet sein, während das Trägermaterial 18 außenhandseitig jeweils nur auf der oberen Hälfte oder dem oberen Drittel des Handschuhs beschichtet ist.

In einer weiteren Abwandlung der Ausführungsbeispiele der Fig. 1 bis 5 kann sich in diesen Figuren die Bezugsziffer 10 auf die Handaußenseite der entsprechenden Handschuhe beziehen, wobei dann die Handinnenseiten (nun nicht gezeigt) nicht mit Mikrokapseln beschichtet sind.

In weiterer Abwandlung der Erfindung kann man die eine Fläche des Handschuhs mit einem ersten mikroverkapselten Material beschichten (z.B. Hautöl) und die andere Fläche des Handschuhs mit einem zweiten mikroverkapselten Material (z.B. Fliegenabwehröl) beschichten. In diesem Falle werden die beiden Seiten des Handschuhs vorzugsweise farblich unterschieden, z.B. unterschiedlich bedrucktes oder unterschiedlich durchgefärbtes Trägermaterial, unterschiedlich gefärbtes Wandmaterial für die Mikrokapseln oder unterschiedlich Färbung der Füllung zumindest teilweise transluzenter Mikrokapseln.

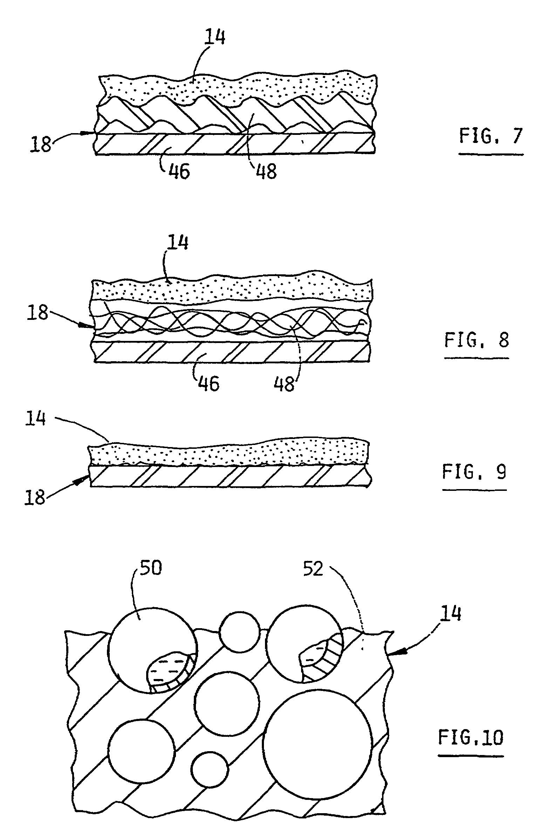

Bei dem in Figur 7 dargestellten Trägermaterial ist auf eine Grundschicht 46 eine Arbeitsschicht 48 aufgebracht. Die Grundschicht 46 besteht aus einer flexiblen dünnen Kunststoffolie, welche für den Inhalt der Mikrokapseln undurchlässig ist. Die Arbeitsschicht 48 ist eine Kunststoffolie, welche durch mechanische und/oder thermische Behandlung gewellt ist, so daß sie eine größere Oberfläche erhält und ähnlich wie ein Faserflor einen flauschigen Griff aufweist. Die Arbeitsschicht 48 ist durch thermische Behandlung oder unter Verwendung einer dünnen Kleberschicht auf der Grundschicht 46 angebracht. Über der Arbeitsschicht 48 liegt die Beschichtung 14, welche durch Aufrakeln aufgebracht ist.

Das Ausführungsbeispiel nach Figur 8 entspricht weitgehend demjenigen nach Figur 7, nur findet als Arbeitsschicht 48 ein Vlies Verwendung.

Beim Ausführungsbeispiel nach Figur 9 ist das Trägermaterial 18 eine einzige Schicht, die schon von Hause aus eine griffige Außenfläche aufweist. Es kann sich hierbei z.B. um eine durch Koronaentladung auf der Außenseite aufgerauhte Kunststoffolie handeln. Statt dessen kann man auch ein dichtes schweißbares Vlies verwenden, welches z.B. zu 70 % aus PP und zu 30 % aus CV besteht.

Vorzugsweise liegt das Flächengewicht der oben beschriebenen Trägermaterialien insgesamt unter 100 g/m², vorzugsweise im Bereich zwischen 60 und 80 g/m². Derartige Materialien sind gut flexibel und lassen sich auch zwischen Zehen und Fingern gut applizieren.

Wie aus Figur 10 ersichtlich, enthält die Beschichtung 14 Mikrokapseln 50 unterschiedlichen Durchmessers, welche durch ein schwaches, sprödes Bindemittel 52 in der Schicht zusammengehalten sind. Unter den Mikrokapseln 50 sind zusätzlich solche mit großer Wandstärke und kleiner Wandstärke, so daß die Mikrokapseln z.T. schon bei leichtem Druck, z.T. bei mittlerem Druck und z.T. erst bei Anwendung hoher Druckes zerbrechen.

Falls gewünscht, kann man auch unterschiedliche Flüssigkeiten in die Mikrokapseln unterschiedlicher mechanischer Festigkeit einbauen, z.B. in leicht zerbrechliche Mikrokapseln ein Öl zum Verschließen von Hautporen, in schwerer zerbrechliche Mikrokapseln ein Fliegenabwehrmittel, welches an der Hautoberfläche wirksam sein soll.

## Patentansprüche

1. Mikrokapselbeschichtetes (14) flexibles Trägermaterial (18), **dadurch gekennzeichnet, daß** das Trägermaterial (18) als Handschuh (12; 20; 30; 40) ausgebildet ist und die Mikrokapselbeschichtung auf der Handschuhaußenseite trägt.

2. Trägermaterial nach Anspruch 1, **dadurch gekennzeichnet,**
**daß** es die Form eines Fausthandschuhs (20) hat.

3. Trägermaterial nach Anspruch 1, **dadurch gekennzeichnet,**
**daß** der Handschuh die Form einer eine ganze Hand aufnehmenden rechteckigen Tasche (30) hat.

4. Trägermaterial nach Anspruch 1, **dadurch gekennzeichnet,**
**daß** es die Form eines rechteckigen Handschuhs (42) hat, der über Zeige-, Mittel- , Ringfinger und kleinen Finger ziehbar ist und oberhalb des Daumenansatzes endet.

5. Trägermaterial nach einem der Ansprüche 1 bis 4, **dadurch gekennzeichnet, daß** der Handschuh ein Zuziehmittel (22) aufweist.

6. Trägermaterial nach einem der Ansprüche 1 bis 5, **dadurch gekennzeichnet, daß** der Handschuh zusätzlich ein Mittel zum Ankleben (34) an der Haut aufweist.

7. Trägermaterial nach Anspruch 5 oder 6, **dadurch gekennzeichnet, daß** das Zuziehmittel (22) und das Mittel zum Ankleben an der Haut (34) gemeinsam durch eine abgewinkelte Lasche (32) gebildet sind.

8. Trägermaterial nach einem der Ansprüche 1 bis 7, **dadurch gekennzeichnet, daß** die aufgebrachte Mikrokapsel-Beschichtung (14) nicht mehr als die oberen zwei Drittel einer oder beider der Flächen des Handschuhs bedeckt.

9. Trägermaterial nach einem der Ansprüche 1 bis 8, **dadurch gekennzeichnet, daß** die Mikrokapsel-Beschichtung (14) auf der Handinnenseite (10) des Handschuhs aufgebracht ist.

10. Trägermaterial nach einem der Ansprüche 1 bis 9, **dadurch gekennzeichnet, daß** die Mikrokapsel-Beschichtung (14) auf der Handaußenseite des Handschuhs aufgebracht ist.

11. Trägermaterial nach einem der Ansprüche 1 bis 10, **dadurch gekennzeichnet, daß** auch das fingerseitige Ende des Handschuhs offen ist.

12. Trägermaterial nach einem der Ansprüche 1 bis 11, **dadurch gekennzeichnet, daß** die die Außenseite des Handschuhs bildende Oberfläche aufgerauht oder flauschig ist.

13. Trägermaterial nach Anspruch 12, **dadurch gekennzeichnet, daß** es ein Verbundmaterial aus einer für den Inhalt der Mikrokapseln undurchlässigen dünnen inneren Grundschicht (46) und einer aufgerauhten oder flauschigen Arbeitsschicht (48) ist.

14. Trägermaterial nach einem der Ansprüche 1 bis 13, **dadurch gekennzeichnet, daß** die Mikrokapseln einen die Haut reizenden Wirkstoff, z.B. einen Wirkstoff zur lokalen Erhöhung der Durchblutung, wie Bienengift oder dergleichen, enthalten.

15. Trägermaterial nach einem der Ansprüche 1 bis 14, **dadurch gekennzeichnet, daß** die Mikrokapseln (50) einen schlecht riechenden Wirkstoff, z.B. einen Fliegenabwehrwirkstoff enthalten.

16. Trägermaterial nach einem der Ansprüche 1 bis 15, **dadurch gekennzeichnet, daß** die Mikrokapseln (50) ein Gemisch aus Mikrokapseln unterschiedlicher Beständigkeit sind, welche sich bezüglich ihres Wandmateriales und/oder bezüglich ihrer Wanddicke unterscheiden.

## Claims

1. A flexible backing material (18) coated with microcapsules (14), **characterised in that** the backing material (18) takes the form of a glove (12; 20; 30; 40) and carries the microcapsule coating on the outside of the glove.

2. A backing material according to claim 1, **characterised in that** it takes the form of a mitten (20).

3. A backing material according to claim 1, **characterised in that** the glove takes the form of a rectangular pouch (30) accommodating a whole hand.

4. A backing material according to claim 1, **characterised in that** it takes the form of a rectangular glove (42), which may be pulled over the fore-, middle, ring and little finger and ends above the base of the thumb.

5. A backing material according to one of claims 1 to 4, **characterised in that** the glove comprises a tightening means (22).

6. A backing material according to one of claims 1 to 5, **characterised in that** the glove additionally comprises a means (34) for sticking it to the skin.

7. A backing material according to claim 5 or claim 6, **characterised in that** the tightening means (22) and the means (34) for sticking the glove to the skin jointly take the form of an angular tab (32).

8. A backing material according to one of claims 1 to 7, **characterised in that** the applied microcapsule coating (14) covers no more than the upper two-thirds of one or both surfaces of the glove.

9. A backing material according to one of claims 1 to 8, **characterised in that** the microcapsule coating (14) is applied to the palm side (10) of the glove.

10. A backing material according to one of claims 1 to 9, **characterised in that** the microcapsule coating (14) is applied to the back of the glove.

11. A backing material according to one of claims 1 to 10, **characterised in that** the finger end of the glove is also open.

12. A backing material according to one of claims 1 to 11, **characterised in that** the surface forming the outside of the glove is roughened or fluffy.

13. A backing material according to claim 12, **characterised in that** it is a composite material of a thin, inner base layer (46), impermeable to the contents of the microcapsules, and a roughened or fluffy working layer (48).

14. A backing material according to one of claims 1 to 13, **characterised in that** the microcapsules contain an active agent which is irritating to the skin, e.g. an active agent for locally increasing circulation, such as bee venom or the like.

15. A backing material according to one of claims 1 to 14, **characterised in that** the microcapsules (50) contain a malodorous active agent, e.g. a fly repellent.

16. A backing material according to one of claims 1 to 15, **characterised in that** the microcapsules (50) are a mixture of microcapsules of varying resistance, which differ in wall material and/or in wall thickness.

## Revendications

1. Matériau support souple (18) revêtu de microcapsules (14), **caractérisé en ce que** le matériau support (18) est conçu sous forme d'un gant (12 ; 20; 30; 40) et **en ce que** le revêtement à base de microcapsules est appliqué sur la face extérieure du gant.

2. Matériau support selon la revendication 1, **caractérisé en ce qu'**il a la forme d'un gant de type mitaine (20).

3. Matériau support selon la revendication 1, **caractérisé en ce que** le gant se présente sous la forme d'une poche rectangulaire (30) accueillant une main dans sa totalité.

4. Matériau support selon la revendication 1, **caractérisé en ce qu'**il présente la forme d'un gant rectangulaire (42) qui peut être enfilé sur l'index, le majeur, l'annulaire et l'auriculaire et qui se termine au-dessus de la base du pouce.

5. Matériau support selon l'une des revendications 1 à 4, **caractérisé en ce que** le gant comporte un moyen de serrage (22).

6. Matériau support selon l'une des revendications 1 à 5, **caractérisé en ce que** le gant comporte en outre un moyen d'adhésion à la peau (34).

7. Matériau support selon la revendication 5 ou 6, **caractérisé en ce que** le moyen de serrage (22) et le moyen d'adhésion à la peau (34) sont formés conjointement par une attache à angle (32).

8. Matériau support selon l'une des revendications 1 à 7, **caractérisé en ce que** le revêtement appliqué à base de microcapsules (14) ne couvre pas plus des deux tiers supérieurs d'une ou des deux surfaces du gant.

9. Matériau support selon l'une des revendications 1 à 8, **caractérisé en ce que** le revêtement à base de microcapsules (14) est appliqué sur la face intérieure de la main (10) du gant.

10. Matériau support selon l'une des revendications 1 à 9, **caractérisé en ce que** le revêtement à base de microcapsules (14) est appliqué sur la face extérieure de la main du gant.

11. Matériau support selon l'une des revendications 1 à 10, **caractérisé en ce que** l'extrémité côté doigts du gant est également ouverte.

12. Matériau support selon l'une des revendications 1 à 11, **caractérisé en ce que** la surface formant la face extérieure du gant est rugueuse ou veloutée.

13. Matériau support selon la revendication 12, **caractérisé en ce qu'**il est un matériau composite composé d'une mince couche de fond interne imperméable (46) au contenu des microcapsules et d'une couché de travail rugueuse ou veloutée (48).

14. Matériau support selon l'une des revendications 1 à 13, **caractérisé en ce que** les microcapsules contiennent un principe actif stimulant la peau, par ex. un principe actif pour augmenter localement la circulation sanguine, tel qu'un venin d'abeilles, ou équivalent.

15. Matériau support selon l'une des revendications 1 à 14, **caractérisé en ce que** les microcapsules (50) contiennent un principe actif malodorant, par exemple un agent répulsif contre les mouches.

16. Matériau support selon l'une des revendications 1 à 15, **caractérisé en ce que** les microcapsules (50) contiennent un mélange de microcapsules de différente résistance, qui se distinguent entre elles de par le matériau de leurs parois et/ou l'épaisseur de leurs parois.
